# EUROPÄISCHE PATENTSCHRIFT

(11) **EP 2 743 342 B1**
(45) Veröffentlichungstag und Bekanntmachung des Hinweises auf die Patenterteilung: **12.07.2017**
(21) Anmeldenummer: 13192709.7
(22) Anmeldetag: 13.11.2013
(51) Int. Cl.: C12N 5/071

(54) **Altershautmodell**
Aged skin model
Modèle de peau mature

(30) Priorität: 13.12.2012 DE 102012223061
(43) Veröffentlichungstag der Anmeldung: 18.06.2014
(73) Patentinhaber: Henkel AG & Co. KGaA, 40589 Düsseldorf (DE)
(72) Erfinder: Brenneisen, Peter, 50769 Köln (DE); Fromm, Johanna, 40589 Düsseldorf (DE); Alili, Lirija, 40225 Düsseldorf (DE); Holtkötter, Olaf, 50354 Hürth (DE); Giesen, Melanie, 47608 Geldern (DE)

(56) Entgegenhaltungen:
- EP-A1- 1 046 402
- WO-A1-99/45770
- J. GRILLARI ET AL: "Contributions of DNA interstrand cross-links to aging of cells and organisms", NUCLEIC ACIDS RESEARCH, Bd. 35, Nr. 22, 26. November 2007 (2007-11-26), Seiten 7566-7576, XP055096308, ISSN: 0305-1048, DOI: 10.1093/nar/gkm1065
- YI-CHAU HUANG ET AL: "Investigation of Mitomycin-C-treated Fibroblasts in 3-D Collagen Gel and Conditioned Medium for Keratinocyte Proliferation", ARTIFICIAL ORGANS, Bd. 30, Nr. 3, 1. März 2006 (2006-03-01), Seiten 150-159, XP055096183, ISSN: 0160-564X, DOI: 10.1111/j.1525-1594.2006.00201.x

## Beschreibung

Die vorliegende Erfindung betrifft ein Verfahren zur Herstellung eines gealterten Hautmodells, insbesondere eines Dermisäquivalentes und eines Ganzhautäquivalentes, sowie die Verwendung nach dem Verfahren hergestellter Altershautäquivalente zur Testung von Substanzen, insbesondere in Bezug auf eine potentiell sensibilisierende Wirkung, bevorzugt auf die Haut.

Rekonstruierte Hautmodelle werden schon seit langem eingesetzt, um Untersuchungen hinsichtlich zellulärer Interaktionen, Penetrationsverhalten von Substanzen, dem Irritationspotenzial von Stoffen sowie der Auswirkung Umwelteinflüsse (z.B. UV Strahlung) auf die Haut durchzuführen. Auch kosmetische Wirkstoffe werden hinsichtlich Ihrer Effektivität z.B. als Anti-Aging Wirkstoffe untersucht. Dabei finden in der Regel Modelle Anwendung, die aus einem epidermalen Teil mit Keratinozyten und einem dermalen Teil mit Fibroblasten bestehen. Aufgrund ihrer einfachen Handhabung und Isolierung werden hier oft sehr junge Fibroblasten aus Vorhaut eingesetzt, wie beispielsweise in der WO2000/029553 A1 oder EP 0 020 753 A1 offenbart.

Die WO99/45770 A1 offenbart gealterte Hautäquivalente, die unter Verwendung von alten Fibroblasten und/oder Keratinocyten oder Zellen hergestellt wurden, welche eine hohe Zahl von Passagen durchlaufen haben.

Grillari et al. offenbaren in Nucelic Acid Res. (2007) 35: 7566-7576 , daß die Verwendung von DNA-Vernetzern wie beispielsweise Mitomycin C mit vorzeitigen Alterungsprozessen in Verbindung gebracht wird. Allerdings wird diese Wirkung als unerwünschte Nebenwirkung im Rahmen einer Chemotherapie geschildert.

Diese unterscheiden sich jedoch essenziell von Fibroblasten aus gealterter Haut, die verschiedene Zeichen der Seneszenz aufweisen. So verringert sich bei gealterten Fibroblasten die Fähigkeit zur Kollagensynthese, collagenabbauende Enzyme wie MMP1 werden induziert, und die Zellteilungsfähigkeit sinkt. Auch in der Epidermis kommt es zu altersbedingten biochemischen Veränderungen, wie z.B. einer Verringerung von Komponenten des Natural Moisturizing Factors (z.B. Filaggin), was im Alter zu einer sehr trockenen schuppigen Haut führen kann (Xerose). Darüber hinaus verändert sich die Lipidzusammensetzung sowie -organisation im Stratum Corneum, wodurch die Barrierewirkung der Haut geschwächt wird.

Darüber hinaus wurde bereits gezeigt, dass das Alter der Fibroblastenspender bei der zellulären Antwort auf bestimmte Wirkstoffe, wie beispielsweise Retinsäure eine Rolle zu spielen scheint. Des Weiteren wurde gezeigt, dass die Migrationsfähigkeit von älteren Fibroblasten, die essenziell für Wundheilungsvorgänge ist, signifikant gegenüber jüngeren Zellen Verringert ist.

Für die Prüfung der Wirksamkeit von Kosmetika, aber auch für die Prüfung des Irritationspotenzials von Reizsubstanzen ist es besonders vorteilhaft, wenn die Zellkulturmodelle den besonderen physiologischen Vorraussetzungen von gealterten Zellen Rechnung tragen. So ist es durchaus möglich dass sich junge Fibroblasten noch zur Stimulierung der Kollagensynthese durch eine Prüfsubstanz anregen lassen, ältere jedoch nicht, da gealterte Zellen einen anderen Stoffwechsel als jüngere Zellen vorweisen. Damit besonders gezielt hochwirksame Produkte speziell für die Altershaut entwickelt werden können, wäre die Entwicklung eines Altershautmodells von Vorteil.

In der Fachliteratur sind Verfahren zur Herstellung von Hautmodellen mit Fibroblasten aus unterschiedlichen Altersstufen bekannt, jedoch bestünde selbst dann, wenn man ausschließlich ältere Fibroblasten einsetzte, der technische Nachteil, dass ältere Zellen sich nicht mehr ausreichend teilen und zu wenig Matrix bilden, um eine künstliche Dermisstruktur auszubilden.

Auch replikativ gealterte Fibroblasten, d.h. junge Fibroblasten, die mehrere Zellpassagen durchlaufen haben, weisen Charakteristika von "alten" Fibroblasten aus (verminderte Zellteilung, verminderte Migration). Aber auch diese haben den Nachteil, dass sie sich nicht mehr ausreichend teilen und zu wenig Matrix bilden, um eine künstliche Dermisstruktur auszubilden.

Daher wäre es von Vorteil, zunächst junge Fibroblasten einzusetzen und diese nach Ausbildung der Dermisstuktur im Modell künstlich altern zu lassen. In der Literatur werden mehrere solcher Möglichkeiten, jedoch nur an Monolayerkulturen, beschrieben, wobei die Alterungsprozesse mit Hilfe von H2O2, der Bestrahlung mit UVB, einer Behandlung mit Busulfan oder dem Einsatz von Beryllium induziert wurden.

Es ist bekannt, dass Mitomycin C dazu eingesetzt werden kann, den Zellzyklus zu arretieren, so dass die Zellen sich nicht weiter teilen. In einigen Publikationen wird darauf hingewiesen, dass mit Mitomycin C behandelte Fibroblasten verschiedene Charakteristika von seneszenten Fibroblasten aufweisen.

Keines der Modelle beschreibt jedoch den Einsatz von mit Mitomycin C gealterten Fibroblasten in dreidimensionalen Hautodellen.

Der vorliegenden Erfindung lag die Aufgabe zugrunde, ein Zellkulturmodell, vorzugsweise ein dreidimensionales Hautäquivalent, bereitzustellen, das den besonderen physiologischen Vorraussetzungen von gealterten Zellen Rechnung trägt. Das bereitzustellende Altershautäquivalent sollte es dabei ermöglichen, gezielt hochwirksame Produkte speziell für die Altershaut entwickeln zu können.

Gegenstand der vorliegegenden Erfindung ist in einer ersten Ausführungsform ein Verfahren zur Herstellung eines gealterten Hautäquivalentes, gekennzeichnet durch die Schritte
a) Bereitstellung von dermalen Matrices;
b) Bereitstellung einer Suspension von Zellen in Fibroblastenmedium mit einer Fibroblasten-Konzentration von 50.000 bis 1.500.000 Fibroblasten/ml
c) Äquilibrierung der Matrices mit zellfreiem Fibroblastenmedium;
d) Aufbringen der Suspension aus Schritt b) auf die äquilibrierten Matrices;
e) Kultivierung der Fibroblasten auf der Matrix;
f) Behandlung des kultivierten Dermismodells mit Mitomycin C.

Die dermalen Matrices können nach an sich bekannten Methoden hergestellt werden und sind auch kommerziell verfügbar. Ein Weg zur Herstellung dermaler Matrices besteht in der Gewinnung von schwer löslichem Kollagen aus kollagenhaltigem Gewebe, der Überführung des schwer löslichen Kollagens mittels einer Mischvorrichtung in eine homogene Kollagensuspension und der anschließenden Lyophilisierung der Kollagensuspension unter Ausbildung einer ersten Matrix A. Diese erste Matrix A kann dann einer Vernetzung unterzogen werden, so dass eine eine zweite, mechanisch stabilisierte Matrix B erzeugt wird, auf welcher Fibroblasten und/oder Keratinozyten (optional nacheinander) eingesät und wachsen gelassen werden können.

Überraschenderweise hat sich herausgestellt, dass die in der EP 0 296 078 A1 und die in der EP 1 778 307 A1 beschriebene Matrices sich besonders gut zur Herstellung eines Altershautmodells mit Hilfe des erfindungsgemäßen Verfahrens eignen. Auf diese Schriften wird daher für die Schritte a) bis e) des erfindungsgemäßen Verfahrens ausdrücklich Bezug genommen.

Im erfindungsgemäßen Verfahren werden die dermalen Matrices zunächst mit zellfreiem Fibroblastenmedium äquilibriert und danach mit einer Suspension von Zellen in Fibroblastenmedium mit einer Fibroblasten-Konzentration von 50.000 bis 1.500.000 Fibroblasten/ml behandelt. Durch die Kultivierung entsteht ein Dermismodell, das im letzten Schritt durch die Behandlung mit Mitomycon C gealtert wird.

Bevorzugte Zellkulturmedien sind DMEM (Dulbecco's Modified Eagle Medium), RPMI 1640, M199 und Ham's F12-Medium. Jedoch kann auch jedes andere beliebige Zellkulturmedium verwendet werden, welches die Kultivierung von Fibroblasten ermöglicht. Als Serum wird vorzugsweise fötales Kälberserum (FCS), aber auch NCS sowie Serumersatzprodukte verwendet und als Puffer zum Beispiel Hepes-Puffer. Der pH-Wert der Lösung aus Zellkulturmedium, Puffer und Serum beträgt in bevorzugter Ausführung 6,0 bis 8,0, beispielsweise 6,5 bis 7,5, insbesondere 7,0

Erfindungsgemäß kann das Medium weitere Faktoren, beispielsweise Hormone, Wachstumsfaktoren, Adhäsionsmittel, Antibiotika, Selektionsmittel, Enzyme und Enzyminhibitoren und ähnliche enthalten.

Zur Verbesserung des Keratinozytenwachstums im Epidermisäquivalent können dem Kulturmedium Faktoren zugefügt werden, die im Falle einer Kokultivierung von Fibroblasten und Keratinozyten von den Fibroblasten freigesetzt werden. Beispielsweise kann der konditionierte Kulturüberstand (d.h. der Mediumüberstand von kultivierten Fibroblasten und/oder Kokulturen von Fibroblasten mit anderen Zelltypen, wie z.B. Endothelzellen, Immunzellen oder Keratinozyten) verwendet werden.

Vorzugsweise enthält die Suspension von Zellen in Fibroblastenmedium eine Fibroblasten-Konzentration von 75.000 bis 1.000.000 Fibroblasten/ml, vorzugsweise von 100.000. bis 750.000 Fibroblasten/ml, weiter bevorzugt von 150.000. bis 600.000 Fibroblasten/ml und insbesondere von 200.000. bis 500.000 Fibroblasten/ml. Es ist besonders bevorzugt, wenn in Schritt d) 0,5 bis 2 ml, vorzugsweise 0,75 bis 1,75 ml, weiter bevorzugt 0,8 bis 1,7 ml, noch weiter bevorzugt 0,9 bis 1,6 ml und insbesondere 1 bis 1,5 ml Suspension auf die äquilibrierten Matrices aufgetragen werden.

Die Kutivierung der Fibroblasten in Schritt e) erfolgt vorzugsweise bei 37°C und 5% CO₂ über einen Zeitraum von 5 bis 30 Tagen, vorzugsweise von 6 bis 25 Tagen, weiter bevorzugt von 7 bis 20 Tagen und insbesondere von 8 bis 14 Tagen.

Wie weiter unten beschrieben können durch die Einsaat verschiedener Zellen unterschiedliche Testsysteme der realen Haut nachgebildet werden: Werden zusätzlich zu den Fibroblasten Keratinozyten und/oder Meanozyten eingesät, resultiert ein Vollhautmodell, werden nur Keratinozyten und/oder Melanozyten eingesät resultiert ein Epidermismodell, während die Einsaat von Fibroblasten ohne Keratinozyten zu einem Dermismodell führt. Das erfindungsgemäße Verfahren stellt ein Dermisäquivalent bereit, welches durch den Alterungsschritt f) als Testsystem für die reife Haut hervorragend geeignet ist.

Der Alterungsschritt f) ist auch auf Epidermisäquivalente oder auf Vollhautäquivalente anwendbar, da die Alterung durch Mitomycin C auf zellulärer Ebene erfolgt. Ein weiterer Gegenstand der vorliegenden Erfindung ist daher ein Verfahren zur Herstellung eines gealterten Hautäquivalentes, bei dem ein Hautäquivalent mit Mitomycin C behandelt wird.

Unter Hautmodell im Sinne der vorliegenden Erfindung sind neben Vollhautäquivalenten (auch Ganzhautmodelle genannt) auch Dermis- und Epidermisäquivalente zu verstehen.

Ein weiterer Gegenstand der vorliegenden Erfindung ist ein Verfahren zur Alterung von Hautäquivalenten, dadurch gekennzeichnet, dass ein nicht-gealtertes Hautäquivalent durch Behandlung mit Mitomycin C gealtert wird.

Mit Hilfe des erfindungsgemäßen Verfahrens lassen sich auch gealterte Vollhautäquivalente bereitstellen. Eine besonders geeignete erfindungsgemäße Verfahrensvariante zur Herstellung eines Vollhautäquivalentes ist gekennzeichnet durch die Schritte
a) Herstellung eines gealterten Dermisäquivalents, umfassend die Schritt a) bis f) gemäß Anspruch 1,
b) Einsaat von Keratinocyten und/oder Melanozyten auf das gealterte Dermisäquivalent;
c) Kultivierung der auf dem Dermiäquivalent wachsenden Keratinocyten und/oder Melanozyten bis zur vollständigen Ausbildung des Vollhautäquivalentes.

Die Alterung erfolgt in allen erfindungsgemäßen Verfahrensvarianten durch den Einsatz von Mitomycin C. Mitomycin C wird exakt als [(1a*S*,8*S*,8a*R*,8b*S*)-6-Amino-8a-methoxy-5-methyl-4,7-dioxo-1,1a,2,4,7,8,8a,8b-octahydroazirino [2,3:3,4]pyrrolo[1,2-a]indol-8-ylmethyl]carbamat bezeichnet und besitzt die CAS-Nr. 50-07-7. Andere gebräuchliche Namen sind [6-Amino-8a-methoxy-5-methyl-4,7-dioxo-1,1a,2,4,7,8,8a,8b-octahydroazireno[2',3':3,4]pyrrolo[1,2-*a*]indol-8-yl]methyl carbamat oder {11-Amino-7-methoxy-12-methyl-10,13-dioxo-2,5-diazatetracyclo[7.4.0.0^{2,7}.0^{4,6}]trideca-1(9),11-dien-8-yl}methyl carbamat.
Mitomycine sind eine Familie Aziridin-haltiger naturstoffe, die sich aus *Streptomyces caespitosus* oderr *Streptomyces lavendulae* isolieren lassen. Mitomycin C lässt sich durch folgende Formel beschreiben:

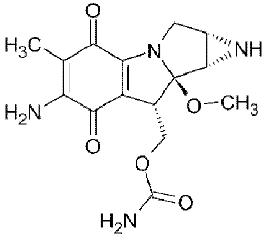

Besonders bevorzugte erfindungsgemäße Verfahren sind dadurch gekennzeichnet, dass die Behandlung des kultivierten Dermisäquivalentes bzw. des Hautäquivalentes mit Mitomycin C mit einer Lösung erfolgt, deren Konzentration an Mitomycin C 10 bis 10.000 nmol/l, vorzugsweise 50 bis 1.000 nmol/l, weiter bevorzugt 100 bis 750 nmol/l und insbesondere 200 bis 500 nmol/l beträgt.
Bei einer molaren Masse des Mitomycins von 334,33 g·mol⁻¹ entspricht 1 Nanomol 0,33433 µg. In anderen Worten sind erfindungsgemäß bevorzugte Verfahren dadurch gekennzeichnet, dass die Behandlung des kultivierten Dermisäquivalentes bzw. des Hautäquivalentes mit Mitomycin C mit einer Lösung erfolgt, deren Konzentration an Mitomycin C 3,34433 µg pro Liter bis 3,3433 mg pro Liter, vorzugsweise 16,7165 bis 334,33 µg pro Liter, weiter bevorzugt 33,433 bis 250,7475 µg pro Liter und insbesondere 66,866 bis 167,165 µg pro Liter beträgt.

Die Einwirkzeit des Mitomycins auf die Zellen bestimmt den Grad der Alterung, wobei lange Einwirkzeiten eine höhere Alterung bewirken. Erfindungsgemäß bevorzugte Verfahren sind dadurch gekennzeichnet, dass die Behandlung des kultivierten Dermisäquivalentes bzw. des Hautäquivalentes mit Mitomycin C über einen Zeitraum von 15 Minuten bis 72 Stunden, vorzugsweise von 1 bis bis 60 Stunden, weiter bevorzugt von 6 bis 48 Stunden und insbesondere von 15 bis 30 Stunden erfolgt.

Je nach Konzentration an Mitomycin C tritt nach einer bestimmten Zeit keine weitere Alterung der Zellen mehr ein. Es hat sich für die Qualität der Hautäquivalente als besonders vorteilhaft erwiesen, eine einmalige Behandlung mit einer bestimmten Konzentration nicht durch Konzentrationserhöhung in ihrer Alterung zu steigern, sondern durch eine nachfolgende zweite Behandlung mit derselben Konzentration. Dies führt zu reproduzierbaren Hautäquivalenten sehr guter Qualität.

Erfindungsgemäß bevorzugte Verfahren sind daher gekennzeichnet durch die Schritte
a) Bereitstellung eines kultivierten Dermisäquivalentes und/oder eines Hautäquivalentes;
b) Behandlung des kultivierten Dermisäquivalentes und/oder des Hautäquivalentes mit einer Lösung, deren Konzentration an Mitomycin C 10 bis 10.000 nmol/l, vorzugsweise 50 bis 1.000 nmol/l, weiter bevorzugt 100 bis 750 nmol/l und insbesondere 200 bis 500 nmol/l beträgt, über einen Zeitraum von 15 Minuten bis 48 Stunden, vorzugsweise von 2 bis bis 36 Stunden, weiter bevorzugt von 6 bis 30 Stunden und insbesondere von 18 bis 24 Stunden,
c) erneute (zweite) Behandlung des kultivierten Dermisäquivalentes und/oder des Hautäquivalentes mit einer Lösung, deren Konzentration an Mitomycin C 10 bis 10.000 nmol/l, vorzugsweise 50 bis 1.000 nmol/l, weiter bevorzugt 100 bis 750 nmol/l und insbesondere 200 bis 500 nmol/l beträgt, über einen Zeitraum von 15 Minuten bis 48 Stunden, vorzugsweise von 2 bis bis 36 Stunden, weiter bevorzugt von 6 bis 30 Stunden und insbesondere von 18 bis 24 Stunden.

Die Behandlung des kultivierten Hautquivalents erfolgt vorzugsweise bei 37°C und 5% CO₂ über die genannten Zeiträume.

Aufgrund der Komplexität des hergestellten Hautmodells kann dieses gezielt für verschiedene Fragestellungen der chemisch-pharmazeutischen Industrie und der kosmetischen Industrie verwendet werden. Insbesondere eignet sich das erfindungsgemäß hergestellte Hautäquivalent zur Produktprüfung, beispielsweise im Hinblick auf Wirksamkeit, unerwünschte Nebenwirkungen, beispielsweise Reiz-, Toxizitäts- und Entzündungswirkungen oder Allergie auslösende Wirkungen, oder Verträglichkeit von Substanzen. Dabei kann es sich um Substanzen handeln, die potentielle Verwendung als Arzneimittel, insbesondere als Dermatika, finden sollen, oder um Substanzen, die Bestandteil von Kosmetika sind, oder auch um Konsumgüter, die mit der Haut in Berührung kommen, wie z.B. Waschmittel etc..

Das erfindungsgemäß hergestellte Hautäquivalent kann beispielsweise auch für Studien zur Resorption, zum Transport und/oder zur Penetration von Substanzen verwendet werden. Darüber hinaus eignet es sich auch zur Untersuchung anderer Agenzien (physikalische Größen), wie Licht oder Wärme, Radioaktivität, Schall, elektromagnetische Strahlung, elektrische Felder beispielsweise zur Untersuchung der Phototoxizität, also der schädigenden Wirkung von Licht unterschiedlicher Wellenlänge, auf Zellstrukturen. Das erfindungsgemäß hergestellte Hautäquivalent kann auch zur Untersuchung der Wundheilung eingesetzt werden. Es eignet sich auch zur Untersuchung der Wirkung von Gasen, Aerosolen, Rauch, Stäuben auf die Zellstrukturen bzw. den Stoffwechsel oder die Genexpression.

Die Wirkungen von Substanzen oder Agenzien auf menschliche Haut lassen sich beispielsweise anhand der Freisetzung von Stoffen, beispielsweise Cytokinen oder Mediatoren, durch Zellen des humanen oder tierischen Hautmodellsystems, sowie der Wirkungen auf Genexpression, Stoffwechsel, Proliferation, Differenzierung und Reorganisation dieser Zellen ermitteln. Unter Verwendung von Verfahren zur Quantifizierung der Zellschädigung, insbesondere unter Verwendung eines Vitalfarbstoffes, wie eines Tetrazoliumderivates, können beispielsweise cytotoxische Wirkungen auf Hautzellen nachgewiesen werden. Die Tests von Substanzen oder Agenzien an dem erfindungsgemäßen humanen Hautäquivalent können sowohl histologische Verfahren als auch immunologische und/oder molekularbiologische Verfahren umfassen.

Eine bevorzugte Ausführungsform der Erfindung umfasst daher ein Verfahren zur Produktprüfung, insbesondere im Hinblick auf Wirksamkeit, unerwünschte Nebenwirkungen, Reiz-, Toxizitäts- und Entzündungswirkungen oder Allergie auslösende Wirkungen, oder Verträglichkeit von Substanzen, umfassend die Schritte
(i) Bereitstellung eines Altershautäquivalentes, umfassend die Schritte
   a) Bereitstellung von dermalen Matrices;
   b) Bereitstellung einer Suspension von Zellen in Fibroblastenmedium mit einer Fibroblasten-Konzentration von 50.000 bis 1.500.000 Fibroblasten/ml
   c) Äquilibrierung der Matrices mit zellfreiem Fibroblastenmedium;
   d) Aufbringen der Suspension aus Schritt b) auf die äquilibrierten Matrices;
   e) Kultivierung der Fibroblasten auf der Matrix;
   f) Behandlung des kultivierten Dermismodells mit Mitomycin C
   g) gegebenenfalls Einsaat von Keratinocyten und/oder Melanozyten auf das Dermisäquivalent; und Kultivierung der auf dem Dermiäquivalent wachsenden Keratinocyten und/oder Melanozyten bis zur vollständigen Ausbildung des Vollhautäquivalentes
(ii) Produktprüfung.

Eine weitere bevorzugte Ausführungsform der Erfindung umfasst ein Verfahren zur Wirksamkeitsbeurteilung von Hautkosmetika, bei dem man
i) ein Altershautäquivalent, herstellt mittels der Schritte
   a) Bereitstellung von dermalen Matrices;
   b) Bereitstellung einer Suspension von Zellen in Fibroblastenmedium mit einer Fibroblasten-Konzentration von 50.000 bis 1.500.000 Fibroblasten/ml
   c) Äquilibrierung der Matrices mit zellfreiem Fibroblastenmedium;
   d) Aufbringen der Suspension aus Schritt b) auf die äquilibrierten Matrices;
   e) Kultivierung der Fibroblasten auf der Matrix;
   f) Behandlung des kultivierten Dermismodells mit Mitomycin C
   g) gegebenenfalls Einsaat von Keratinocyten und/oder Melanozyten auf das Dermisäquivalent; und Kultivierung der auf dem Dermiäquivalent wachsenden Keratinocyten und/oder Melanozyten bis zur vollständigen Ausbildung des Vollhautäquivalentes
ii) die Anwesenheit, Menge, Expression und/oder Aktivität mindestens eines Parameters aus der Gruppe der Prokollagene, Kollagene, Elastin, Fibronectin, Hyaluronsäure, Glucosaminoglucane, Matrixmetalloproteinasen sowie Differenzierungsmarker der Epidermis (z.B. Involucrin, Filaggrin, Transglutaminase, Loricrin) des Altershautäquivalents misst,

i) das Altershautäquivalent mit einem topisch applizierten Hautkosmetikum kosmetisch behandelt,
ii) die Anwesenheit, Menge, Expression und/oder Aktivität mindestens eines Parameters aus der Gruppe der Prokollagene, Kollagene, Elastin, Fibronectin, Hyaluronsäure, Glucosaminoglucane, Matrixmetalloproteinasen sowie Differenzierungsmarker der Epidermis (z.B. Involucrin, Filaggrin, Transglutaminase, Loricrin) des nunmehr behandelten Altershautäquivalentes misst,
iii) den Wert aus Schritt i) mit dem Werte aus Schritt iv) vergleicht und iv) die Wirksamkeit des Hautkosmetikums anhand des Einflusses auf die Anwesenheit, Menge, Expression und/oder Aktivität mindestens eines Parameters aus der Gruppe der Prokollagene, Kollagene, Elastin, Fibronectin, Hyaluronsäure, Glucosaminoglucane, Matrixmetalloproteinasen sowie Differenzierungsmarker der Epidermis (z.B. Involucrin, Filaggrin, Transglutaminase, Loricrin) bewertet.

Bezüglich bevorzugter Ausführungsformen der erfindungsgemäßen Verfahren gilt mutatis mutandis das zu den erstgenannten erfindungsgemäßen Verfahren Gesagte.

### Beispiele:

### Beispiel 1: Herstellung eines gealterten Dermismodells

Als dermale Matrix wird die in der EP 0 296 078 A1 und die in der EP 1 778 307 A1 beschriebene Matrix eingesetzt. Vor der Aussaat auf die vernetzten Collagen-Matrices werden Fibroblasten einer geeigneten Passage in Zellkulturflaschen mit Fiborblastenmedium vorkultiviert. Nach Erreichen der gewünschten Zelldichte wird das Kulturmedium abgesaugt. Die Zellen werden durch Zugabe einer Trypsinlösung in die Kulturflaschen vom Gefässboden abgelöst, mit Kulturmedium gewaschen und abzentrifugiert. Nach Bestimmung der Zellzahl wird die Zellsuspension auf eine Konzentration von 1 - 6 X 10⁵ Fibroblasten/ml eingestellt.

Aus den Vertiefungen der Mikrotiterplatte, die die mit Fibroblastenmedium äquilibrierten Matrices enthalten, wird das Medium so weit abgesaugt, das die Matrices feucht bleiben. Auf die Oberfläche der Matrices wird jeweils 1 ml Fibroblastenmedium, enthaltend jeweils 1 - 6 X 10⁵ Fibroblasten, pipettiert, ohne die Oberfläche dabei zu verletzen. Nach Beendigung der Einsaat wird der Plattendeckel aufgesetzt und die Platte waagerecht in den Inkubator gestellt. Die Kultivierung der Fibroblasten auf der Matrix erfolgt bei 37°C und 5 % CO₂. Das Kulturmedium wird in regelmässigen Intervallen gewechselt. Nach 2-4 Wochen ist das Dermismodell fertig entwickelt und es erfolgt die Mitomycin C Behandlung für 1h bis 48h in einer Konzentration von 100-500 µM zur Induktion der Alterung.

### Beispiel 2: Herstellung eines gealterten Vollhautmodells

Als dermale Matrix wird die in der EP 0 296 078 A1 und die in der EP 1 778 307 A1 beschriebene Matrix eingesetzt. Vor der Aussaat auf die vernetzten Collagen-Matrices werden Fibroblasten einer geeigneten Passage in Zellkulturflaschen mit Fibroblastenmedium vorkultiviert. Nach Erreichen der gewünschten Zelldichte wird das Kulturmedium abgesaugt. Die Zellen werden durch Zugabe einer Trypsinlösung in die Kulturflaschen vom Gefässboden abgelöst, mit Fibroblastenmedium gewaschen und abzentrifugiert. Nach Bestimmung der Zellzahl wird die Zellsuspension auf eine Konzentration von 1 - 6 X 105 Fibroblasten/ml eingestellt.

Aus den Vertiefungen der Mikrotiterplatte, die die mit Kulturmedium äquilibrierten Matrices enthalten, wird das Medium so weit abgesaugt, das die Oberfläche der Matrices feucht bleiben. Auf die Oberfläche der Matrices wird jeweils 1 ml Fibroblastenmedium, enthaltend jeweils 1 - 6 X 105 Fibroblasten, pipettiert, ohne die Oberfläche dabei zu verletzen. Nach Beendigung der Einsaat wird der Plattendeckel aufgesetzt und die Platte waagerecht in den Inkubator gestellt. Die Kultivierung der Fibroblasten auf der Matrix erfolgt bei 37°C und 5% CO₂. Das Kulturmedium wird in regelmäßigen Intervallen gewechselt. Nach einer Kultivierungszeit der Fibroblasten von 2-4 Wochen erfolgt die Mitomycin C Behandlung für 1h bis 48h in einer Konzentration von 100-500 µM.

Nach der Behandlung mit Mitomycin C werden die Keratinocyten auf das Dermismodell ausgesät. Dazu werden Keratinocyten einer geeigneten Passage in Zellkulturflaschen mit Keratinocytenmedium vorkultiviert. Nach Erreichen der gewünschten Zelldichte wird das Kulturmedium abgesaugt. Die Zellen werden durch Zugabe einer Trypsinlösung in die Kulturflaschen vom Gefässboden abgelöst, mit Kulturmedium gewaschen und abzentrifugiert. Nach Bestimmung der Zellzahl wird die Zellsuspension auf eine Konzentration von 2 - 4 X 10⁵ Keratinocyten/ml eingestellt. Aus den Vertiefungen der Mikrotiterplatte, die die Dermisäquivalente enthalten, wird das Medium so weit abgesaugt, dass die Oberfläche der Dermisäquivalente feucht bleibt. Auf die Oberfläche der Dermismodelle wird jeweils 1 ml Keratinocytenmedium, enthaltend jeweils 2 - 6 X 10⁵ Keratinocyten, pipettiert, ohne die Oberfläche dabei zu verletzen. Nach Beendigung der Einsaat wird der Plattendeckel aufgesetzt und die Platte waagerecht in den Inkubator gestellt (Submerskultur). Die Kultivierung der Fibroblasten und Keratinocyten auf der Matrix erfolgt 7 Tage bei 37°C und 5% CO₂. Das Kulturmedium wird in regelmässigen Intervallen gewechselt.

Nach Ablauf der 2-7-tägigen Submerskultur wird das Nährmedium aus den Vertiefungen abgesaugt. Die noch unfertigen Vollhautmodelle werden aus den Vertiefungen herausgeholt und auf Filterpapiere gesetzt. Die Filterpapiere liegen auf metallenen Abstandhaltern jeweils in einer Petrischale. Nachdem die unfertigen Vollhautmodelle auf dem Filterpapier platziert sind, wird die Petrischale mit Kulturmedium (Airlift-Medium; Air-Liquid-Interface-Medium) so weit gefüllt, dass das Medium den oberen Rand des Filterpapiers erreicht und sich um die Basis der Hautmodelle verteilt. Die Oberfläche der Hautmodelle wird nicht von Kulturmedium bedeckt (Airlift-Kultur oder Air-Liquid-Interface). In Abhängigkeit vom gewünschten Differenzierungsgrad werden die Hautmodelle 1-4 Wochen in der Air-Liquid-Interface belassen. Das Kulturmedium wird in regelmäßigen Intervallen gewechselt.

### Beispiel 3: Größenvermessung zum Nachweis der Seneszenz

Gealterte Fibroblasten weisen gegenüber jungen Fibroblasten eine deutlich Veränderte Morphologie auf. Junge Fibroblasten sind kleiner und weisen eine Spindelform auf. Gealterte Fibroblasten sind vergrößert, zeigen ausgefranste Ränder und verlieren ihre Spindelform. Veränderungen in der Zellgröße sind also ein Zeichen von Seneszenz. Um nachzuweisen, dass die mit Mitomycin C behandelten Fibroblasten im Modell gealterten Fibroblasten ähneln, wurden sie nach 3-wöchiger Inkubation im Air-Liquid-Interface mittels Collagenasebehandlung wieder aus dem Modell herausgelöst und in definierter Zellzahl in Petrischalen gesät. Nach 24h erfolgte mittels Photodkumentation und Bildanalyseverfahren die Größenbestimmung. Als Kontrollen wurden junge, ebenfalls in einem Vollhautmodell kultivierte Fibroblasten mitgeführt. Die Tabelle 1 zeigt die Ergebnise der Zellgrößenvermessung, dabei wurde die Kontrolle mit jungen Fibroblasten =1 gesetzt.

**Tab 1: Größenbestimmung verschiedener Fibroblasten**

| Zellsorte im Modell | Größe | SD [%] |
|---|---|---|
| Junge Fibroblasten | 1 | 0,6 |
| Mitomycin C behandelt | 4,3 | 2,2 |

Die Ergebnisse zeigen, dass die mit Mitomycin C behandelten Fibroblasten deutlich größer sind als die jungen Fibroblasten.

### Beispiel 4: Nachweis der Seneszenz durch SA-ß-Galactosidase

Der der seneszenz-assoziierte Biomarker SA-ß-Galactosidase wird nur in gealterten Zellen exprimiert und ist daher ein Parameter zur Bestimmung der Zellalterung. Zur Visualisierung erfolgt der Nachweis über eine Umsetzung von X-Gal (5-Brom-4-chlor-3-indoxyl-β-D-galactopyranosid) durch die Seneszenz-assoziierte β-Galaktosidase bei pH6 und anschließender Oxidation des Produkts durch Luftsauerstoff, wodurch tiefblauer Indigo-Farbstoff entsteht. Zum Nachweis der Seneszenz werden mit Mitomycin C behandelte Fibroblasten 3-wöchiger Inkubation im Air-Liquid-Interface mittels Collagenasebehandlung wieder aus dem Modell herausgelöst und in definierter Zellzahl in Petrischalen gesät. Nach 24h erfolgte der Nachweis SA- ß- galactosidase wie oben beschrieben. Als Kontrollen wurden junge, ebenfalls in einem Vollhautmodell kultivierte Fibroblasten mitgeführt.

Bei den durch Mitomycin C gealterten Fibroblasten lässt sich eine deutliche Blaufärbung erkennen, was den seneszenten Status der Zellen belegt.

### Beispiel 5: Nachweis der Collagenneubildung

Dermale Fibroblasten produzieren eine collgenreiche extrazelluläre Matrix, die der Haut mechanische Stabilität und Widerstandsfähigkeit verleiht. In gealterter Haut sinkt die Kollagenproduktion der Fibroblasten, während die Fragmentierung und der Abbau von Collagenfasern durch Matrixmetalloproteinasen (MMPs). Zum Nachweis der Collagenneusynthese wurden neugebildete Collagenfasern in einem durch Mitomycin C Behandlung gealterten Vollhautmodell mittels so genannter Azanfärbung nachgewiesen. Die Azan-Färbung ist eine histologische Übersichtsfärbung. Der Name setzt sich aus den beiden Farbkomponenten Azokarmin G und Anilinblau-Goldorange zusammen. Mit der Azan-Färbung färben sich Zellkerne und das Zytoplasma rot, sowie Collagenfasern blau. Als Kontrolle dienten Vollhautmodelle mit jungen Fibroblasten.

Die Untersuchungsergebnisse zeigen deutlich, dass die Collagenneusynthese (blaue Fasern) in gealterten Modellen reduziert ist.

### Beispiel 6: Nachweis von Matrixmetalloproteinase

Wie in Beispiel 5 beschrieben steigt die Fragmentierung der Collgenfasern in gealterter Haut an. Ursache hierfür sind Matrixmetalloproteinasen (MMPs), die in gealterter Haut eine gesteigerte Aktivität aufweisen. Zum Nachweis von MMP1 wurde der Kulturüberstand von Vollhautmodellen mit jungen Fibroblasten und altersinduzierten Fibroblasten mittels eines kommerziell erhältlichen Enzymimmumnoassays (ELISA) hinsichtlich des Gehalts von MMP1 untersucht. In Tabelle 2 sind Ergebnisse zusammengefasst. Dabei wurde der Gehalt an MMP1 in "jungen" Vollhautmodellen = 100% gesetzt.

**Tab. 2 Gehalt an MMP1 in Vollhautmodellen**

| Vollhautmodell | MMP1 MW [%] | SD [%] |
|---|---|---|
| jung | 100 | 5 |
| gealtert | 219 | 33 |
| p-value *(student t-test)* | 0,0036 | |

Die Ergebnisse zeigen, dass der Gehalt an MMP1 in gealterten Modellen signifikant erhöht ist gegenüber der Kontrolle mit jungen Fibroblasten. Dies belegt, ebenso wie die Beispiele 3-5, dass das Modell mit Mitomycin C behandelten Fibroblasten viele Merkmale gealterter Haut aufweist und damit besser für die gezielte Entwicklung von Anti-Aging-Kosmetikprodukten geeignet ist, als die bisher im Stand der Technik dokumentierten Systeme.

## Patentansprüche

1. Verfahren zur Herstellung eines gealterten Hautäquivalentes, **gekennzeichnet durch** die Schritte
a) Bereitstellung von dermalen Matrices;
b) Bereitstellung einer Suspension von Zellen in Fibroblastenmedium mit einer Fibroblasten-Konzentration von 50.000 bis 1.500.000 Fibroblasten/ml
c) Äquilibrierung der Matrices mit zellfreiem Fibroblastenmedium;
d) Aufbringen der Suspension aus Schritt b) auf die äquilibrierten Matrices;
e) Kultivierung der Fibroblasten auf der Matrix;
f) Behandlung des kultivierten Dermismodells mit Mitomycin C.

2. Verfahren zur Herstellung eines gealterten Hautäquivalentes, **dadurch gekennzeichnet, dass** ein Hautäquivalent mit Mitomycin C behandelt wird.

3. Verfahren zur Alterung von Hautäquivalenten, **dadurch gekennzeichnet, dass** ein nicht-gealtertes Hautäquivalent durch Behandlung mit Mitomycin C gealtert wird.

4. Verfahren zur Herstellung eines Vollhautäquivalentes, **gekennzeichnet durch** die Schritte
a) Herstellung eines gealterten Dermisäquivalents, umfassend die Schritt a) bis f) gemäß Anspruch 1,
b) Einsaat von Keratinocyten und/oder Melanozyten auf das gealterte Dermisäquivalent;
c) Kultivierung der auf dem Dermiäquivalent wachsenden Keratinocyten und/oder Melanozyten bis zur vollständigen Ausbildung des Vollhautäquivalentes.

5. Verfahren nach einem der Ansprüche 1 bis 4, **dadurch gekennzeichnet, dass** die Behandlung des kultivierten Dermisäquivalentes bzw. des Hautäquivalentes mit Mitomycin C mit einer Lösung erfolgt, deren Konzentration an Mitomycin C 10 bis 10.000 nmol/l, vorzugsweise 50 bis 1.000 nmol/l, weiter bevorzugt 100 bis 750 nmol/l und insbesondere 200 bis 500 nmol/l beträgt.

6. Verfahren nach einem der Ansprüche 1 bis 5, **dadurch gekennzeichnet, dass** die Behandlung des kultivierten Dermisäquivalentes bzw. des Hautäquivalentes mit Mitomycin C über einen Zeitraum von 15 Minuten bis 72 Stunden, vorzugsweise von 1 bis bis 60 Stunden, weiter bevorzugt von 6 bis 48 Stunden und insbesondere von 15 bis 30 Stunden erfolgt.

7. Verfahren nach einem der Ansprüche 1 bis 6, **gekennzeichnet durch** die Schritte
a) Bereitstellung eines kultivierten Dermisäquivalentes und/oder eines Hautäquivalentes;
b) Behandlung des kultivierten Dermisäquivalentes und/oder des Hautäquivalentes mit einer Lösung, deren Konzentration an Mitomycin C 10 bis 10.000 nmol/l, vorzugsweise 50 bis 1.000 nmol/l, weiter bevorzugt 100 bis 750 nmol/l und insbesondere 200 bis 500 nmol/l beträgt, über einen Zeitraum von 15 Minuten bis 48 Stunden, vorzugsweise von 2 bis bis 36 Stunden, weiter bevorzugt von 6 bis 30 Stunden und insbesondere von 18 bis 24 Stunden,
c) erneute (zweite) Behandlung des kultivierten Dermisäquivalentes und/oder des Hautäquivalentes mit einer Lösung, deren Konzentration an Mitomycin C 10 bis 10.000 nmol/l, vorzugsweise 50 bis 1.000 nmol/l, weiter bevorzugt 100 bis 750 nmol/l und insbesondere 200 bis 500 nmol/l beträgt, über einen Zeitraum von 15 Minuten bis 48 Stunden, vorzugsweise von 2 bis bis 36 Stunden, weiter bevorzugt von 6 bis 30 Stunden und insbesondere von 18 bis 24 Stunden.

8. Verfahren zur Produktprüfung, insbesondere im Hinblick auf Wirksamkeit, unerwünschte Nebenwirkungen, Reiz-, Toxizitäts- und Entzündungswirkungen oder Allergie auslösende Wirkungen, oder Verträglichkeit von Substanzen, umfassend die Schritte
(i) Bereitstellung eines Altershautäquivalentes, umfassend die Schritte
a) Bereitstellung von dermalen Matrices;
b) Bereitstellung einer Suspension von Zellen in Fibroblastenmedium mit einer Fibroblasten-Konzentration von 50.000 bis 1.500.000 Fibroblasten/ml
c) Äquilibrierung der Matrices mit zellfreiem Fibroblastenmedium;
d) Aufbringen der Suspension aus Schritt b) auf die äquilibrierten Matrices;
e) Kultivierung der Fibroblasten auf der Matrix;
f) Behandlung des kultivierten Dermismodells mit Mitomycin C
g) gegebenenfalls Einsaat von Keratinocyten und/oder Melanozyten auf das Dermisäquivalent; und Kultivierung der auf dem Dermiäquivalent wachsenden Keratinocyten und/oder Melanozyten bis zur vollständigen Ausbildung des Vollhautäquivalentes
(ii) Produktprüfung.

9. Verfahren zur Wirksamkeitsbeurteilung von Hautkosmetika, **dadurch gekennzeichnet, dass** man
i) ein Altershautäquivalent, herstellt mittels der Schritte
a) Bereitstellung von dermalen Matrices;
b) Bereitstellung einer Suspension von Zellen in Fibroblastenmedium mit einer Fibroblasten-Konzentration von 50.000 bis 1.500.000 Fibroblasten/ml
c) Äquilibrierung der Matrices mit zellfreiem Fibroblastenmedium;
d) Aufbringen der Suspension aus Schritt b) auf die äquilibrierten Matrices;
e) Kultivierung der Fibroblasten auf der Matrix;
f) Behandlung des kultivierten Dermismodells mit Mitomycin C
g) gegebenenfalls Einsaat von Keratinocyten und/oder Melanozyten auf das Dermisäquivalent; und Kultivierung der auf dem Dermiäquivalent wachsenden Keratinocyten und/oder Melanozyten bis zur vollständigen Ausbildung des Vollhautäquivalentes
ii) die Anwesenheit, Menge, Expression und/oder Aktivität mindestens eines Parameters aus der Gruppe der Prokollagene, Kollagene, Elastin, Fibronectin, Hyaluronsäure, Glucosaminoglucane, Matrixmetalloproteinasen sowie Differenzierungsmarker der Epidermis (z.B. Involucrin, Filaggrin, Transglutaminase, Loricrin) des Altershautäquivalents misst,
iii) das Altershautäquivalent mit einem tropisch applizierten Hautkosmetikum kosmetisch behandelt,
iv) die Anwesenheit, Menge, Expression und/oder Aktivität mindestens eines Parameters aus der Gruppe der Prokollagene, Kollagene, Elastin, Fibronectin, Hyaluronsäure, Glucosaminoglucane, Matrixmetalloproteinasen sowie Differenzierungsmarker der Epidermis (z.B. Involucrin, Filaggrin, Transglutaminase, Loricrin) des nunmehr behandelten Altershautäquivalentes misst,
v) den Wert aus Schritt i) mit dem Werte aus Schritt iv) vergleicht und
vi) die Wirksamkeit des Hautkosmetikums anhand des Einflusses auf die Anwesenheit, Menge, Expression und/oder Aktivität mindestens eines Parameters aus der Gruppe der Prokollagene, Kollagene, Elastin, Fibronectin, Hyaluronsäure, Glucosaminoglucane, Matrixmetalloproteinasen sowie Differenzierungsmarker der Epidermis (z.B. Involucrin, Filaggrin, Transglutaminase, Loricrin) bewertet.

## Claims

1. A method for producing an aged skin equivalent, **characterized by** the steps of
a) providing dermal matrices;
b) providing a suspension of cells in a fibroblast medium having a fibroblast concentration of from 50,000 to 1,500,000 fibroblasts/ml
c) equilibrating the matrices using a cell-free fibroblast medium;
d) applying the suspension from step b) to the equilibrated matrices;
e) culturing the fibroblasts on the matrix;
f) treating the cultured dermis model with mitomycin C.

2. The method for producing an aged skin equivalent, **characterized in that** a skin equivalent is treated with mitomycin C.

3. A method for aging skin equivalents, **characterized in that** a non-aged skin equivalent is aged by treating with mitomycin C.

4. A method for producing a full skin equivalent, **characterized by** the steps of
a) producing an aged dermis equivalent using steps a) to f) according to claim 1,
b) sowing keratinocytes and/or melanocytes onto the aged dermis equivalent;
c) culturing the keratinocytes and/or melanocytes growing on the dermis equivalent until the full skin equivalent has fully formed.

5. The method according to one of claims 1 to 4, **characterized in that** the cultured dermis equivalent and/or the skin equivalent is treated with mitomycin C using a solution in which the concentration of mitomycin C is from 10 to 10,000 nmol/l, preferably from 50 to 1,000 nmol/l, more preferably from 100 to 750 nmol/l and in particular from 200 to 500 nmol/l.

6. The method according to one of claims 1 to 5, **characterized in that** the cultured dermis equivalent and/or the skin equivalent is treated with mitomycin C over a period of from 15 minutes to 72 hours, preferably from 1 to 60 hours, more preferably from 6 to 48 hours and in particular from 15 to 30 hours.

7. The method according to one of claims 1 to 6, **characterized by** the steps of
a) providing a cultured dermis equivalent and/or a skin equivalent;
b) treating the cultured dermis equivalent and/or the skin equivalent with the solution in which the concentration of mitomycin C is from 10 to 10,000 nmol/l, preferably from 50 to 1,000 nmol/l, more preferably from 100 to 750 nmol/l and in particular from 200 to 500 nmol/l, over a period of from 15 minutes to 48 hours, preferably from 2 to 36 hours, more preferably from 6 to 30 hours and in particular from 18 to 24 hours,
c) treating the cultured dermis equivalent and/or the skin equivalent again (for a second time) with a solution in which the concentration of mitomycin C is from 10 to 10,000 nmol/l, preferably from 50 to 1,000 nmol/l, more preferably from 100 to 750 nmol/l and in particular from 200 to 500 nmol/l, over a period of from 15 minutes to 48 hours, preferably from 2 to 36 hours, more preferably from 6 to 30 hours and in particular from 18 to 24 hours.

8. A method for testing the product, in particular in relation to effectiveness, undesired side effects, irritant, toxicological and inflammatory effects or allergenic effects, or compatibility of substances, comprising the steps of
(i) providing an aged skin equivalent using the steps of
a) providing dermal matrices;
b) providing a suspension of cells in a fibroblast medium having a fibroblast concentration of 50,000 to 1,500,000 fibroblasts/ml
c) equilibrating the matrices using a cell-free fibroblast medium;
d) applying the suspension from step b) to the equilibrated matrices;
e) culturing the fibroblasts on the matrix;
f) treating the cultured dermis model with mitomycin C
g) optionally sowing keratinocytes and/or melanocytes onto the dermis equivalent, and culturing the keratinocytes and/or melanocytes growing on the dermis equivalent until the full skin equivalent has fully formed
(ii) testing the product.

9. A method for assessing the effectiveness of skin cosmetics, **characterized in that**
i) an aged skin equivalent is produced using the steps of
a) providing dermal matrices;
b) providing a suspension of cells in a fibroblast medium having a fibroblast concentration of from 50,000 to 1,500,000 fibroblasts/ml
c) equilibrating the matrices using a cell-free fibroblast medium;
d) applying the suspension from step b) to the equilibrated matrices;
e) culturing the fibroblasts on the matrix;
f) treating the cultured dermis model with mitomycin C
g) optionally sowing keratinocytes and/or melanocytes onto the dermis equivalent; and culturing the keratinocytes and/or melanocytes growing on the dermis equivalent until the full skin equivalent has fully formed
ii) the presence, amount, expression and/or activity of at least one parameter from the group of procollagens, collagens, elastin, fibronectin, hyaluronic acid, glucosaminoglucanes, matrix metalloproteinases and differentiation markers of the epidermis (e.g. involucrin, filaggrin, transglutaminase, loricrin) of the aged skin equivalent are measured,
iii) the aged skin equivalent is cosmetically treated with a topically applied skin cosmetic,
iv) the presence, amount, expression and/or activity of at least one parameter from the group of procollagens, collagens, elastin, fibronectin, hyaluronic acid, glucosaminoglucanes, matrix metalloproteinases and differentiation markers of the epidermis (e.g. involucrin, filaggrin, transglutaminase, loricrin) of the now treated aged skin equivalent are measured,
v) the value from step i) is compared with the value from step iv) and
vi) the effectiveness of the skin cosmetic is evaluated on the basis of the influence on the presence, amount, expression and/or activity of at least one parameter from the group of procollagens, collagens, elastin, fibronectin, hyaluronic acid, glucosaminoglucanes, matrix metalloproteinases and differentiation markers of the epidermis (e.g. involucrin, filaggrin, transglutaminase, loricrin).

## Revendications

1. Procédé de fabrication d'un équivalent de peau vieillie, **caractérisé par** les étapes de :
a) Préparation d'une matrice dermique ;
b) Préparation d'une suspension de cellules dans un milieu de culture de fibroblastes avec une concentration en fibroblastes de 50.000 à 1.500.000 fibroblastes/ml
c) Réarrangement de la matrice avec un milieu de culture de fibroblastes sans cellules ;
d) Application de la suspension de l'étape b) sur la matrice réarrangée ;
e) Culture des fibroblastes sur la matrice ;
f) Traitement du modèle dermique cultivé à la mitomycine C.

2. Procédé de fabrication d'un équivalent de peau vieillie, **caractérisé en ce qu'**on traite un équivalent de la peau avec de la mitomycine C.

3. Procédé de vieillissement d'équivalents de la peau, **caractérisé en ce qu'**on vieillit un équivalent de la peau non-vieillie par traitement à la mitomycine C.

4. Procédé de fabrication d'un équivalent de peau complète, **caractérisé par** les étapes de :
a) Fabrication d'un équivalent de peau vieillie, comprenant les étapes a) à f) selon la revendication 1,
b) Insémination de kératinocytes et/ou de mélanocytes sur l'équivalent de peau vieillie ;
c) Culture des kératinocytes et/ou mélanocyte en croissance sur l'équivalent de peau jusqu'à formation complète de l'équivalent de peau complète.

5. Procédé selon une des revendications 1 à 4, **caractérisé en ce que** le traitement de l'équivalent de peau cultivée ou resp. de l'équivalent de peau à la mitomycine C est fait avec une solution dont la concentration en mitomycine C est de 10 à 10.000 nmol/l, de préférence 50 à 1.000 nmol/l, plus préférentiellement 100 à 750 nmol/l et en particulier 200 à 500 nmol/l.

6. Procédé selon une des revendications 1 à 5, **caractérisé en ce que** le traitement de l'équivalent de peau cultivé ou resp. de l'équivalent de peau à la mitomycine C est fait sur une durée de 15 minutes à 72 heures, de préférence de 1 à 60 heures, plus préférentiellement de 6 à 48 heures et en particulier de 15 à 30 heures.

7. Procédé selon une des revendications 1 à 6, **caractérisé par** les étapes de :
a) Préparation d'un équivalent de peau cultivé et/ou d'un équivalent de peau ;
b) Traitement de l'équivalent de peau cultivé et/ou de l'équivalent de peau avec une solution dont la concentration en mitomycine C est de 10 à 10.000 nmol/l, de préférence 50 à 1.000 nmol/l, plus préférentiellement 100 à 750 nmol/l et en particulier 200 à 500 nmol/l, sur une durée de 15 minutes à 48 heures, de préférence de 2 à 36 heures, plus préférentiellement de 6 à 30 heures et en particulier de 18 à 24 heures,
c) Nouveau (deuxième) traitement de l'équivalent de peau cultivé et/ou de l'équivalent de peau avec une solution dont la concentration en mitomycine C est de 10 à 10.000 nmol/l, de préférence 50 à 1.000 nmol/l, plus préférentiellement 100 à 750 nmol/l et en particulier 200 à 500 nmol/l, sur une durée de 15 minutes à 48 heures, de préférence de 2 à 36 heures, plus préférentiellement de 6 à 30 heures et en particulier de 18 à 24 heures.

8. Procédé de contrôle de produit, en particulier en termes d'efficacité, d'effets secondaires indésirables, d'induction d'irritation, de toxicité, d'inflammation ou d'allergie, ou de compatibilité avec des substances, comprenant les étapes de :
(i) Préparation d'un équivalent de peau vieillie, comprenant les étapes de :
a) Préparation d'une matrice dermique ;
b) Préparation d'une suspension de cellules dans un milieu de culture de fibroblastes avec une concentration en fibroblastes de 50.000 à 1.500.000 fibroblastes/ml ;
c) Réarrangement de la matrice avec un milieu de culture de fibroblastes sans cellules ;
d) Application de la suspension de l'étape b) sur la matrice réarrangée ;
e) Culture des fibroblastes sur la matrice ;
f) Traitement du modèle dermique cultivé à la mitomycine C
g) Éventuellement, insémination de kératinocytes et/ou de mélanocytes sur l'équivalent de peau vieillie ; et culture des kératinocytes et/ou mélanocyte en croissance sur l'équivalent de peau jusqu'à formation complète de l'équivalent de peau complète
(ii) Contrôle de produit.

9. Procédé d'évaluation d'efficacité de cosmétiques dermatologiques, **caractérisé par** :
i) un équivalent de peau vieillie, confectionné grâce aux étapes de :
a) Préparation de matrices dermiques ;
b) Préparation d'une suspension de cellules dans un milieu de culture de fibroblastes avec une concentration en fibroblastes de 50.000 à 1.500.000 fibroblastes/ml ;
c) Réarrangement de la matrice avec un milieu de culture de fibroblastes sans cellules ;
d) Application de la suspension de l'étape b) sur la matrice réarrangée ;
e) Culture des fibroblastes sur la matrice ;
f) Traitement du modèle dermique cultivé à la mitomycine C ;
g) Éventuellement, insémination de kératinocytes et/ou de mélanocytes sur l'équivalent de peau vieillie ; et culture des kératinocytes et/ou mélanocyte en croissance sur l'équivalent de peau jusqu'à formation complète de l'équivalent de peau complète ;
ii) une mesure de la présence, de la quantité, de l'expression et/ou de l'activité d'au moins un paramètre dans le groupe du procollagène, du collagène, de l'élastine, de la fibronectine, de l'acide hyaluronique, des glycosaminoglycanes, des métalloprotéases matricielles ainsi que des marqueurs de différentiation de l'épiderme (ex. involucrine, filaggrine, transglutaminases, loricrine) de l'équivalent de peau vieillie,
iii) un traitement de l'équivalent de peau vieillie avec un cosmétique en application tropique,
iv) un mesure de la présence, la quantité, l'expression et/ou l'activité d'au moins un paramètre dans le groupe du procollagène, du collagène, de l'élastine, de la fibronectine, de l'acide hyaluronique, des glycosaminoglycanes, des métalloprotéases matricielles ainsi que des marqueurs de différentiation de l'épiderme (ex. involucrine, filaggrine, transglutaminases, loricrine) de l'équivalent de peau vieillie traité
v) une comparaison de la valeur de l'étape i) avec la valeur de l'étape iv), et
vi) une évaluation de l'efficacité du cosmétique dermatologique sous l'influence de la présence, la quantité, l'expression et/ou l'activité d'au moins un paramètre dans le groupe du procollagène, du collagène, de l'élastine, de la fibronectine, de l'acide hyaluronique, des glycosaminoglycanes, des métalloprotéases matricielles ainsi que des marqueurs de différentiation de l'épiderme (ex. involucrine, filaggrine, transglutaminases, loricrine).
